# EUROPEAN PATENT APPLICATION

(11) **EP 4 725 937 A1**
(43) Date of publication of application: **15.04.2026**
(21) Application number: 24206346.9
(22) Date of filing: 14.10.2024
(51) Int. Cl.: C07C 233/59, C07C 233/60, C07C 323/40, A61K 31/165

(54) **PROCESS FOR THE SYNTHESIS OF BICYCLIC CARBOXAMIDE COMPOUNDS**

(71) Applicant: Acousia Therapeutics GmbH, 72070 Tübingen (DE)
(72) Inventor: Van Zeist, Abko, 6002 WD Weert (NL); Eggels, Denise, 6071 LM Swalmen (NL); Toronto, Dawn, Bend, 97703 (US)
(74) Representative: Patentanwälte Ruff, Wilhelm, Beier, Dauster & Partner mbB

(57) **Abstract**

The present invention discloses a process for the synthesis of a compound according to formula I or a pharmaceutically acceptable salt thereof 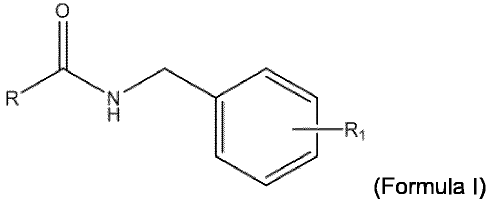 wherein
- R is a bicycloalkyl group, preferably an unsubstituted bicycloalkyl group.

The process the following steps, preferably performed sequential in time,
I. asymmetric ring opening of a carbic anhydride, preferably cis-5-Norbornene-endo-2,3-dicarboxylic anhydride or (3aR,4S,7R,7aS)-3a,4,7,7a-tetrahydro-4,7-methanoisobenzofuran-1,3-dione, yielding (1S,2R,3S,4R)-3-(methoxycarbonyl)bicyclo[2.2.1]hept-5-ene-2-carboxylic acid,
II. epimerizing of (1S,2R,3S,4R)-3-(methoxycarbonyl)bicyclo[2.2.1]hept-5-ene-2-carboxylic acid yielding (1S,2R,3R,4R)-3-(methoxycarbonyl)bicyclo[2.2.1]hept-5-ene-2-carboxylic acid,
III. hydrogenating (1S,2R,3R,4R)-3-(methoxycarbonyl)bicyclo[2.2.1]hept-5-ene-2-carboxylic acid yielding (1R,2R,3R,4S)-3-(methoxycarbonyl)bicyclo[2.2.1]heptane-2-carboxylic acid,
IV. decarboxylating (1R,2R,3R,4S)-3-(methoxycarbonyl)bicyclo[2.2.1]heptane-2-carboxylic acid yielding methyl (1S,2S,4R)-bicyclo[2.2.1]heptane-2-carboxylate,
V. hydrolysing methyl (1S,2S,4R)-bicyclo[2.2.1]heptane-2-carboxylate yielding (1S,2S,4R)-bicyclo[2.2.1]heptane-2-carboxylic acid,
VI. coupling (1S,2S,4R)-bicyclo[2.2.1]heptane-2-carboxylic acid with a benzylamine, preferably substituted with a substituent selected from the group consisting of heteroatoms, in particular F, SF₅, CF₃ or OCF₃, or an alkyl group, in particular an ethyl-, methyl- or propyl-group, yielding a compound according to formula I, and
VII. optionally crystallising of the compound according to formula I.

## Description

### TECHNICAL FIELD AND PRIOR ART

The present invention relates to a novel process for the synthesis of bicyclic carboxamide compounds, in particular useful as pharmaceutically active agents. The present invention also relates to a pharmaceutical composition or medicament comprising these bicyclic carboxamide compounds produced by this process, the use of this pharmaceutical composition or medicament in the prevention or treatment of inner ear diseases and a crystalline form of a bicyclic carboxamide compound, preferably obtained by a process according to the inventive process.

The reaction conditions for bicyclic carboxamide compounds often require multiple solvents in one reaction which makes the workup challenging and laborious. Further, the use of solvents such as dichloromethane, dimethylformamide (DMF) and diethyl ether is often used irrespective of their unsafe environmental, teratogenic and flammable and explosive characteristics.

Furthermore, known workup conditions are typical scaled-up laboratory procedures, preferably including for example concentration to dryness steps, which are not fit for application in a fixed production equipment used in a larger scale production.

Finally, when it comes to the synthesis of a bicyclic moiety a sufficient yield and chiral purity are especially difficult to obtain.

The present invention addresses all of these points, as the inventive process allows reliable and safe synthetic procedures which are fit to be executed in fixed production equipment. Furthermore, the use of undesired solvents such as dichloromethane (harmful to the environment), DMF (teratogenic, risk for formation of N-nitrosamines) and diethyl ether (extremely flammable and explosive) is avoided.

### SUMMARY OF THE INVENTION

In view of the foregoing, the object underlying the present invention is therefore to make available a novel process for the synthesis of bicyclic carboxamide compounds which properly addresses the above-mentioned need.

The above object and further objects are solved by a process according to claim 1, a pharmaceutical composition comprising a compound obtained by this process according to claim 13, a pharmaceutical composition for use in in the prevention or treatment of inner ear diseases according to claim 14 and a crystalline form of a compound according to formula I according to claim 15. Preferred embodiments of the invention are defined in the dependent claims and in the present description. The subject-matter and wording, respectively, of all claims is hereby incorporated into the description by explicit reference.

According to a first aspect, the present invention relates to a process for the synthesis of a compound according to formula I,

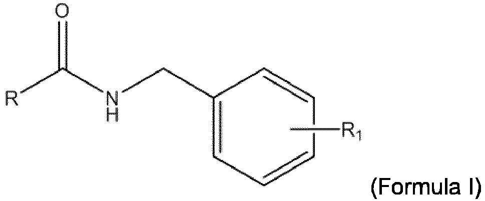

In formula I R is a bicycloalkyl group. Preferably, in formula I R is a substituted or unsubstituted bicycloalkyl group. More preferably in formula I R is an unsubstituted bicycloalkyl group. Preferably, the compound according to formula I is a bicyclic compound.

Preferably, in formula I R₁ is a substituent selected from the group consisting of heteroatoms, in particular F, SF₅, CF₃ or OCF₃, or an alkyl group, in particular an ethyl-, methyl- or propyl-group.

The process for the synthesis of a compound according to formula I comprises or consists of the following steps, preferably performed sequential in time,
I. asymmetric ring opening of a carbic anhydride, preferably cis-5-Norbornene-endo-2,3-dicarboxylic anhydride or (3aR,4S,7R,7aS)-3a,4,7,7a-tetrahydro-4,7-methanoisobenzofuran-1,3-dione, yielding (1S,2R,3S,4R)-3-(methoxycarbonyl) bicyclo[2.2.1]hept-5-ene-2-carboxylic acid,
II. epimerizing of (1S,2R,3S,4R)-3-(methoxycarbonyl)bicyclo[2.2.1]hept-5-ene-2-carboxylic acid yielding (1S,2R,3R,4R)-3-(methoxycarbonyl)bicyclo[2.2.1]hept-5-ene-2-carboxylic acid,
III. hydrogenating (1S,2R,3R,4R)-3-(methoxycarbonyl)bicyclo[2.2.1]hept-5-ene-2-carboxylic acid yielding (1R,2R,3R,4S)-3-(methoxycarbonyl)bicyclo[2.2.1]heptane-2-carboxylic acid,
IV. decarboxylating (1R,2R,3R,4S)-3-(methoxycarbonyl)bicyclo[2.2.1]heptane-2-carboxylic acid yielding methyl (1S,2S,4R)-bicyclo[2.2.1]heptane-2-carboxylate,
V. hydrolysing methyl (1S,2S,4R)-bicyclo[2.2.1]heptane-2-carboxylate yielding (1S,2S,4R)-bicyclo[2.2.1]heptane-2-carboxylic acid, and
VI. coupling (1S,2S,4R)-bicyclo[2.2.1]heptane-2-carboxylic acid with a benzylamine , preferably with 3-(pentafluoro-λ6-sulfaneyl)phenyl)methanamine, yielding a compound according to formula I. Preferably, the benzylamine is substituted with a substituent selected from the group consisting of heteroatoms, in particular F, SF₅, CF₃ or OCF₃, and/or alkyl groups, in particular an ethyl-, methyl- or propyl-group. Further preferred, the benzylamine is 3-(pentafluoro-λ6-sulfaneyl)phenyl)methanamine.

Preferably, the process further comprises a step VII., crystallising of a compound according to formula I, preferably performed after step VI., more preferably performed directly after step VI.

Further preferred, in step VII. a compound according to formula I, is crystallised, preferably from cyclohexane or ethyl acetate and n-heptane.

Preferably, the inventive route of the synthesis as described above, comprises or consists of a six step, or optional seven step, linear synthesis, of which five steps, in particular steps I. to V., are required for the synthesis of the chiral building block (1S,2S,4R)-bicyclo[2.2.1]heptane-2-carboxylic acid. In other words, the synthetic route preferably starts with the preparation of chiral intermediate (1S,2R,3S,4R)-3-(methoxycarbonyl)bicyclo[2.2.1]hept-5-ene-2-carboxylic acid by asymmetric ring opening of a carbic anhydride, preferably cis-5-Norbornene-endo-2,3-dicarboxylic anhydride or (3aR,4S,7R,7aS)-3a,4,7,7a-tetrahydro-4,7-methanoisobenzofuran-1,3-dione, preferably using methanol in the presence of (+)-quinidine to induce asymmetry. This material is then epimerized, preferably under influence of sodium methoxide with methanol as solvent, yielding (1S,2R,3R,4R)-3-(methoxycarbonyl)bicyclo[2.2.1]hept-5-ene-2-carboxylic acid. (1S,2R,3R,4R)-3-(methoxycarbonyl)bicyclo[2.2.1]hept-5-ene-2-carboxylic acid is then hydrogenated, preferably with hydrogen gas in the presence of palladium on charcoal catalyst in ethyl acetate, which results in the formation of (1R,2R,3R,4S)-3-(methoxycarbonyl)bicyclo[2.2.1]heptane-2-carboxylic acid. This compound is decarboxylated in the next step, preferably in a Barton decarboxylation converting (1R,2R,3R,4S)-3-(methoxycarbonyl)bicyclo[2.2.1]heptane-2-carboxylic acid into its acid chloride with oxalyl chloride and decarboxylating (1R,2R,3R,4S)-3-(methoxycarbonyl)bicyclo[2.2.1]heptane-2-carboxylic acid with sodium pyrithione and tert-butyl mercaptan in toluene at a temperature between 100°C and 105°C, yielding methyl (1S,2S,4R)-bicyclo[2.2.1]heptane-2-carboxylate. Afterwards, methyl (1S,2S,4R)-bicyclo[2.2.1]heptane-2-carboxylate is hydrolysed, preferably with 1M sodium hydroxide in methanol, to form the penultimate (1S,2S,4R)-bicyclo[2.2.1]heptane-2-carboxylic acid. This compound is coupled with a benzylamine, preferably the benzylamine is 3-(pentafluoro-λ6-sulfaneyl)phenyl)methanamine, preferably with propylphosphonic anhydride (T3P) as coupling reagent and ethyl acetate as solvent. This gives the desired compound according to formula I, which is preferably crystallised from cyclohexane or ethyl acetate/n-heptane.

The term "bicycloalkyl" as used according to the present invention means a group of substances consisting of ring-shaped, saturated hydrocarbons. A distinction is made between bonded (condensed) bicycloalkyl groups and bridged bicycloalkyl groups and spiro bicycloalkyl groups. Preferably, the bicycloalykly group in formula I is a bridged bicycloalkyl group.

The term "solvent" as used according to the present invention means an anorganic or organic solvent, preferably an organic solvent, that dissolves a solute, resulting in a solution.

The term "INT2804" as used according to the present inventions means a carbic anhydride, preferably cis-5-Norbornene-endo-2,3-dicarboxylic anhydride or (3aR,4S,7R,7aS)-3a,4,7,7a-tetrahydro-4,7-methanoisobenzofuran-1,3-dione, having the empirical formula C₉H₈O₃, a molecular weight (MW) of 164.05, the CAS number 129-64-6 and the formula II.

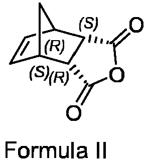

The term "INT2805" as used according to the present inventions means (1S,2R,3S,4R)-3-(methoxycarbonyl)bicyclo[2.2.1]hept-5-ene-2-carboxylic acid, having the empirical formula C₁₀H₁₂O₄, a MW of 196.20, the CAS-number 96243-73-1, and the formula III.

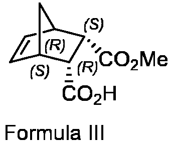

The term "INT2806" as used according to the present inventions means (1S,2R,3R,4R)-3-(methoxycarbonyl)bicyclo[2.2.1]hept-5-ene-2-carboxylic acid, having the empirical formula C₁₀H₁₂O₄, a MW of 196.20, the CAS-number 125226-91-7, and the formula IV.

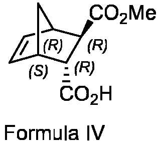

The term "INT2807" as used according to the present inventions means (1R,2R,3R,4S)-3-(methoxycarbonyl)bicyclo[2.2.1]heptane-2-carboxylic acid, having the empirical formula C₁₀H₁₄O₄, a MW of 198.22, the CAS-number 125226-86-0, and the formula V.

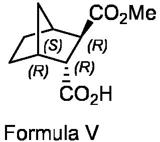

The term "INT2808" as used according to the present inventions means methyl (1S,2S,4R)-bicyclo[2.2.1]heptane-2-carboxylate, having the empirical formula C₉H₁₄O₂, a MW of 154.21, the CAS-number 186182-40-1, and the formula VI.

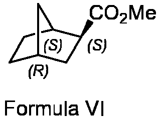

The term "INT2809" as used according to the present inventions means (1S,2S,4R)-bicyclo[2.2.1]heptane-2-carboxylic acid, having the empirical formula C₈H₁₂O₂, a MW of 140.18, the CAS-number 79026-23-6, and the formula VII.

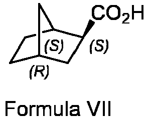

The term "INT2810" as used according to the present inventions means a benzylamine, preferably the benzylamine is 3-(pentafluoro-λ6-sulfaneyl)phenyl)methanamine, having the empirical formula C₇H₈F₅NS, a MW of 233.20, the CAS-number 771573-34-3 and the formula VIII.

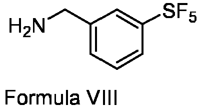

The term "INT2828" as used according to the present inventions means (1R,2R,3R,4S)-bicyclo[2.2.1]hept-5-ene-2,3-dicarboxylic acid, having the empirical formula C₉H₁₀O₄, a MW of 182.18, the CAS-number 1200-88-0 (rel), 32216-02-7 (abs) and the formula IX.

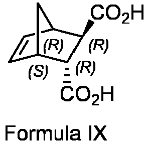

The present invention is based in particular on the surprising findings that
- A reliable and scalable process for the six chemical steps required to prepare a compound according to formula I could be developed.
- Non-scalable and unwanted process steps such as evaporation to dryness and drying over sodium sulfate can be avoided.
- Environmentally hazardous solvents such as dichloromethane, DMF and diethyl ether are replaced by less harmful alternatives.
- The solvent volumes used in the inventive process could be reduced. Leading to a more environment friendly process.
- In step III. and step IV. of the process, preferably no isolation of the product is required. This saves time and increases the yield since developed isolation procedures are laborious and may result in significant product loss.

- A compound according to formula I can be produced in good yield and purity.
- A good chiral purity of the compound according to formula I could be obtained.
- The risk of N-nitrosamine formation was reduced.
- Removal from step I. and IV. of environmentally hazardous dichloromethane, and the ability to perform step IV. (Barton decarboxylation) without DMF, which limits the risk of forming N-nitrosamines.

In an embodiment of the invention in step I. the asymmetric ring opening of a carbic anhydride, preferably cis-5-Norbornene-endo-2,3-dicarboxylic anhydride or (3aR,4S,7R,7aS)-3a,4,7,7a-tetrahydro-4,7-methanoisobenzofuran-1,3-dione, yielding (1S,2R,3S,4R)-3-(methoxycarbonyl)bicyclo[2.2.1]hept-5-ene-2-carboxylic acid is performed with methanol in the presence of (+)-quinidine in toluene, preferably to induce asymmetry.

The temperature in step I. may preferably be of -45°C to -70°C, more preferably -50°C to -65°C, further preferred -55°C to -60°C. The mentioned temperature is considered to be the optimum for obtaining the best chiral induction, a good purity and a short reaction time. If the temperature is lower better chiral purity can be obtained. However, the reaction is much slower at those lower temperatures. It was surprisingly discovered that the above mentioned temperature allows both a good chiral purity and a relatively fast reaction time.

In other words, the synthetic route of the inventive process preferably starts with the preparation of chiral intermediate (1S,2R,3S,4R)-3-(methoxycarbonyl)bicyclo[2.2.1]hept-5-ene-2-carboxylic acid by asymmetric ring opening of a carbic anhydride, preferably cis-5-Norbornene-endo-2,3-dicarboxylic anhydride or (3aR,4S,7R,7aS)-3a,4,7,7a-tetrahydro-4,7-methanoisobenzofuran-1,3-dione, preferably with methanol in the presence of (+)-quinidine, preferably to induce asymmetry.

Further preferred, a concentration of 0,06 M to 0,10 M carbic anhydride, preferably cis-5-Norbornene-endo-2,3-dicarboxylic anhydride or (3aR,4S,7R,7aS)-3a,4,7,7a-tetrahydro-4,7-methanoisobenzofuran-1,3-dione, more preferably 0.08 M, in toluene is used in step I. The mentioned concentration of carbic anhydride, preferably cis-5-Norbornene-endo-2,3-dicarboxylic anhydride or (3aR,4S,7R,7aS)-3a,4,7,7a-tetrahydro-4,7-methanoisobenzofuran-1,3-dione, in toluene is considered to be the optimum for obtaining the best chiral induction.

Asymmetric ring opening is also known as desymmetrisation.

In another embodiment of the invention in step I. the product (1S,2R,3S,4R)-3-(methoxycarbonyl)bicyclo[2.2.1]hept-5-ene-2-carboxylic acid is separated from (+)-quinidine by extractions comprising the following steps, preferably performed sequential in time,
a. adding of phosphoric acid to the reaction mixture to dissolve (+)-quinidine in the aqueous phase,
b. back extraction of the aqueous phase with isopropyl acetate or toluene to increase yield of the product,
c. extraction of (1S,2R,3S,4R)-3-(methoxycarbonyl)bicyclo[2.2.1]hept-5-ene-2-carboxylic acid from the organic phase into the aqueous phase with potassium bicarbonate,
d. acidification of the aqueous phase with phosphoric acid and extraction of the product into the organic phase with an organic solvent, preferably isopropyl acetate or ethyl acetate, and
e. isolation of (1S,2R,3S,4R)-3-(methoxycarbonyl)bicyclo[2.2.1]hept-5-ene-2-carboxylic acid by crystallisation from isopropyl acetate and n-heptane.

Since dichloromethane is an environmental harmful solvent, no emission of dichloromethane is allowed. However, this solvent is often used in the prior art. It is an object of the present invention that this solvent should be replaced. However, usually in the prior art when (+)-quinidine is used in the asymmetric ring opening, it is removed by evaporating the solvent, preferably toluene, and dissolving quinidine in diluted aqueous hydrochloric acid, the product is then extracted with dichloromethane.

Surprisingly, it could be discovered that using phosphoric acid to dissolve (+)-quinidine and back extraction with isopropyl acetate or ethyl acetate, preferably isopropyl acetate, in step I., gives an advantageous scalable and reliable workup procedure, especially in comparison with aqueous hydrochloric acid and back extraction with dichloromethane. Therefore, preferably phosphoric acid and/or isopropyl acetate is used in step I., preferably for the removal of (+)-quinidine. More preferably, aqueous phosphoric acid is used an amount of 1.0 to 3.0 mol/kg, more preferably 2.0 mol/kg (19.6 wt%).

Compared with aqueous hydrochloric acid, using phosphoric acid in step I. the phase separations proceed without a considerable high amount of product left in the aqueous phase. Therefore, preferably isopropyl acetate is chosen as the solvent for the back extraction, instead of dichloromethane. To further speed up of the isopropyl acetate phase separations an intermediate cleaning of the reactor with water and isopropyl acetate, to remove minor amounts of insoluble can be performed.

Preferably, performing step I. as described above (1S,2R,3S,4R)-3-(methoxycarbonyl)bicyclo[2.2.1]hept-5-ene-2-carboxylic acid is obtained in high yield with high LC (liquid chromatography)- and chiral purity. In the developed process, compared to the prior art dichloromethane was replaced with isopropyl acetate or ethyl acetate, preferably isopropyl acetate. Isopropyl acetate or ethyl acetate, preferably isopropyl acetate, has the advantages that it is environmentally friendly and showing a good azeotrope with water (for example 10.6 wt% water in isopropyl acetate), preferably allowing to replace sodium sulfate drying used in the prior art for azeotropic drying.

Diethyl ether is extremely flammable and possesses a risk of forming peroxides. Therefore, it is not allowed to use diethyl ether in a production environment. The use of solvents such as diethyl ether is often used irrespective of their unsafe flammable characteristics.

However, diethyl ether is often used in chemical processes in particular for asymmetric ring opening. Surprisingly, the use of diethyl ether could be avoided in the isolation of the product, preferably (1S,2R,3S,4R)-3-(methoxycarbonyl)bicyclo[2.2.1]hept-5-ene-2-carboxylic acid, preferably when a crystallisation from isopropyl acetate and n-heptane is performed, giving the product in high yield and purity.

In other words, isolation of the product, preferably (1S,2R,3S,4R)-3-(methoxycarbonyl)bicyclo[2.2.1]hept-5-ene-2-carboxylic acid, can be performed by crystallisation from isopropyl acetate and n-heptane, yielding (1S,2R,3S,4R)-3-(methoxycarbonyl)bicyclo[2.2.1]hept-5-ene-2-carboxylic acid in high yield and purity. The use of dichloromethane and diethyl ether can be avoided this way.

Further, since evaporation to dryness is complex in fixed production equipment and scale up processes, a solvent reduction, preferably of toluene, is advantageous for a convenient production process in larger scale and preferably an isolation by crystallisation.

In another embodiment of the invention in step II. the epimerizing of (1S,2R,3S,4R)-3-(methoxycarbonyl)bicyclo[2.2.1]hept-5-ene-2-carboxylic acid is performed with a solution of sodium methoxide in methanol as solvent.

Preferably, the epimerizing of (1S,2R,3S,4R)-3-(methoxycarbonyl)bicyclo[2.2.1]hept-5-ene-2-carboxylic acid comprises the following steps, preferably performed sequential in time,
a. removing of possible hydroxide ions from sodium methoxide by reacting sodium methoxide, preferably with 1. eq., more preferably 0.8 to 1.2 eq., of methyl acetate for at least 16 h, preferably 16 to 20h, at 55 to- 60°C, preferably 50 to 65°C,
b. dissolving of (1S,2R,3S,4R)-3-(methoxycarbonyl)bicyclo[2.2.1]hept-5-ene-2-carboxylic acid in methanol and adding the solution into sodium methoxide, preferably 5.0 eq., more preferably 4.5 to 5.5 eq., sodium methoxide, prepared in step a), and preferably reacting at 65-70°C, preferably 60 to 75°C for 3 h, preferably 2,5 to 3,5h,
c. quenching the reaction mixture, prepared in step b), by using 4 to 6 eq. of formic acid, preferably 5.5 eq. of formic acid and
d. switching the solvent, preferably from methanol, to isopropyl acetate, and extraction of the product, preferably (1S,2R,3R,4R)-3-(methoxycarbonyl)bicyclo[2.2.1]hept-5-ene-2-carboxylic acid into organic phase with organic solvent, preferably isopropyl acetate or ethyl acetate, more preferably isopropyl acetate,
e. optionally isolation of (1S,2R,3R,4R)-3-(methoxycarbonyl)bicyclo[2.2.1]hept-5-ene-2-carboxylic acid by crystallisation from n-heptane and cyclohexane.

The term "eq." and "equivalent" as used according to the present inventions means molar equivalent, i.e. equivalent in mol, regarding the ratio of the respective compound. For example, reacting sodium methoxide with 1. eq. of methyl acetate, means reacting sodium methoxide with methyl acetate in a molar ratio of 1:1.

Surprisingly it could be discovered that, a higher chiral purity is obtained with more equivalents of sodium methoxide, and for an advantageous conversion a higher temperature and a longer reaction time is important, as described above. The chiral purity decreases only slightly at higher temperatures. Therefore, it could be concluded that high equivalents of methoxide at high temperature, in particular at reflux of the methanol are the most practical conditions for this reaction.

Preferably, step b. of step II is performed using 4.0 to 6.0, preferably 5.0, eq. of sodium methoxide, preferably at reflux temperature. This preferably reduces the reaction time to below 4 hours and racemisation is minimized.

The features described above allow step II. to be a scalable and reliable procedure for the preparation of (1S,2R,3R,4R)-3-(methoxycarbonyl)bicyclo[2.2.1]hept-5-ene-2-carboxylic acid. Usually, epimerizing includes steps that are not fit for scale-up in fixed multi-purpose production equipment. This could be avoided by step II. according to this invention.

Evaporation to dryness is not possible in production equipment. A solvent switch to an appropriate solvent and an extraction procedure needs to be developed for a convenient production process.

By crystallising (1S,2R,3R,4R)-3-(methoxycarbonyl)bicyclo[2.2.1]hept-5-ene-2-carboxylic acid from n-heptane and cyclohexane, evaporation to dryness can be avoided. Furthermore, preferably this crystallisation provides a method for purification of the product.

Preferably, by using isopropyl acetate in step d. of step II., dichloromethane can be avoided in step II.

In the prior art during epimerizing steps, usually aqueous ammonium chloride is added to a reaction mixture containing a considerable amount of sodium methoxide. This may lead to a high pH and the presence of significant amounts of free ammonium hydroxide. Since the handling times in a production environment are much longer than in a laboratory environment, this represents a serious risk of hydrolysis of the product. Neutralisation with a weak acid under anhydrous conditions as described above is preferable.

By using formic acid as described above, sodium sulfate drying may be replaced by azeotropic drying.

In another embodiment of the invention in step III. the hydrogenating of (1S,2R,3R,4R)-3-(methoxycarbonyl)bicyclo[2.2.1]hept-5-ene-2-carboxylic acid is performed with hydrogen gas in the presence of a palladium on charcoal catalyst, preferably in ethyl acetate.

The use of a palladium on charcoal catalyst, preferably in ethyl acetate, preferably allows a simple and reliable procedure for the hydrogenation of (1S,2R,3R,4R)-3-(methoxycarbonyl)bicyclo[2.2.1]hept-5-ene-2-carboxylic acid.

Preferably, in step III. the hydrogenating of (1S,2R,3R,4R)-3-(methoxycarbonyl)bicyclo[2.2.1]hept-5-ene-2-carboxylic acid is performed in 8 to 30 rel. vol, preferably 8 to 12 rel. vol, more preferably 10 rel. vol., of solvent.

The term "rel. vol." or "relative volume" as used according to the present invention means volume of a solvent, defined as the ratio of the mass of solute that is present in a solution, relative to the volume of the solvent. For example, 10 rel. vol. solvent means 10 volumes of the solvent in relation to the amount of the solute.

Surprisingly, it was found that instead of using higher amounts of solvents in step III. like it is usually done in the state of the art, it is possible to reduce the amount of solvents used in step III. as described above and still end up with a simple and reliable procedure for the hydrogenation of (1S,2R,3R,4R)-3-(methoxycarbonyl)bicyclo[2.2.1]hept-5-ene-2-carboxylic acid.

In another embodiment of the invention in step III. the hydrogenating of (1S,2R,3R,4R)-3-(methoxycarbonyl)bicyclo[2.2.1]hept-5-ene-2-carboxylic acid is performed with a catalyst present in an amount from 0,001 % to 0,1 % by weight, preferably from 0,005 % to 0,05 % by weight, more preferably from 0,0075 % to 0,0125 % by weight, based on the total weight of the composition.

Surprisingly, it was found that instead of using higher molarities or amounts of a catalyst in step III. like it is usually done in the state of the art, it is possible to reduce the molarity or amount of a catalyst used in step III. as described above and still end up with simple and reliable procedure for the hydrogenation of (1S,2R,3R,4R)-3-(methoxycarbonyl)bicyclo[2.2.1]hept-5-ene-2-carboxylic acid.

Preferably, in step III. the hydrogenating of (1S,2R,3R,4R)-3-(methoxycarbonyl)bicyclo[2.2.1]hept-5-ene-2-carboxylic acid is performed with a pressure of 2 to 6 barg, more preferably 3 to 4 barg.

Surprisingly, it was found that instead of using a higher pressure in step III. like it is usually done in the state of the art, it is possible to reduce the pressure used in step III. as described above and still end up with simple and reliable procedure for the hydrogenation of (1S,2R,3R,4R)-3-(methoxycarbonyl)bicyclo[2.2.1]hept-5-ene-2-carboxylic acid, that allows the reaction not to proceeded too fast and prevents the temperature during the hydrogenation of step III. to became too high. Preferably, leading to a comparatively purer crude reaction mixture.

Evaporation to dryness is not possible in multi-purpose production equipment. A convenient isolation procedure should be developed. However, crystallisation of the product may lead to a significant loss of a pure reaction product in the mother liquor. According to the analytical data, the crude reaction mixture in step III. is pure enough so the crystallisation of the reaction mixture may be skipped, and the crude reaction mixture can directly be used for step IV.

In other words, preferably step III. is free of an isolation step, preferably crystallisation step, of (1R,2R,3R,4S)-3-(methoxycarbonyl)bicyclo[2.2.1]heptane-2-carboxylic acid.

In another embodiment of the invention in step IV. the decarboxylating of (1R,2R,3R,4S)-3-(methoxycarbonyl)bicyclo[2.2.1]heptane-2-carboxylic acid is performed in a Barton decarboxylation, preferably comprising the following steps, preferably performed sequential in time,
a. solvent switch of ethyl acetate into toluene,
b. converting (1R,2R,3R,4S)-3-(methoxycarbonyl)bicyclo[2.2.1]heptane-2-carboxylic acid into its acid chloride with a chloride compound, preferably oxalyl chloride, thionyl chloride or phosphoryl chloride, more preferably oxalyl chloride and
c. decarboxylating (1R,2R,3R,4S)-3-(methoxycarbonyl)bicyclo[2.2.1]heptane-2-carboxylic acid with sodium pyrithione and tert-butyl mercaptan in 30 to 50 rel. vol, preferably 40 rel. vol., solvent, preferably toluene, preferably at a temperature between 90°C and 120°C, preferably between 100°C and 105°C.

The term "Barton decarboxylation" as used according to the present invention means a radical reaction in which a carboxylic acid is converted to a thiohydroxamate ester (commonly referred to as a Barton ester). The product is then heated in the presence of a radical initiator and a suitable hydrogen donor to afford the decarboxylated product.

Surprisingly, it was found that instead of using higher amounts of solvents in step IV. like it is usually done in the state of the art, it is possible to reduce the amount of solvents used in step IV. as described above and still end up with a simple and reliable procedure for decarboxylating of (1R,2R,3R,4S)-3-(methoxycarbonyl)bicyclo[2.2.1]heptane-2-carboxylic acid. Further, due to the Barton decarboxylation the use of N,N-dimethylformamide (DMF) could be avoided, which is usually used in decarboxylation. Usually, DMF is used for the preparation of acid chloride and could be avoided in step IV.

In another embodiment of the invention in step IV. the product methyl (1S,2S,4R)-bicyclo[2.2.1]heptane-2-carboxylate is used as a solution, preferably in methanol, for the next step of the inventive process.

In other words, preferably step IV. is free of an isolation step, preferably crystallisation step, of (1S,2S,4R)-bicyclo[2.2.1]heptane-2-carboxylate.

Further, preferably step IV. is free of an isolation step, preferably crystallisation step, of (1R,2R,3R,4S)-3-(methoxycarbonyl)bicyclo[2.2.1]heptane-2-carboxylic acid.

Surprisingly, it was found that instead of isolation, in particular isolation by distillation the resulting methanol solution was pure enough to submit it to the next step. Therefore, no isolation, in particular by distillation, may be necessary, which may lead to product loss. Nevertheless, after the decarboxylation stage, impurities may be removed by a basic and an acidic aqueous extraction.

In another embodiment of the invention in step V. the hydrolysing of methyl (1S,2S,4R)-bicyclo[2.2.1]heptane-2-carboxylate is performed with sodium hydroxide in methanol.

Preferably, in step V. the hydrolysing of methyl (1S,2S,4R)-bicyclo[2.2.1]heptane-2-carboxylate is performed with 0.75M to 1.25M, more preferably 1M sodium hydroxide in methanol.

Surprisingly, it was found that instead of using lithium hydroxide, preferably in tetrahydrofuran (THF), more preferably in THF and water, as reagent for the hydrolysis in step IV., like it is usually done in the state of the art, it is possible to replace lithium hydroxide in THF, more preferably in THF and water, by sodium hydroxide in methanol, more preferably in methanol and water. Thereby, lithium hydroxide in THF as an expensive and toxic reagent may be replaced.

In another embodiment of the invention the isolation of the product (1S,2S,4R)-bicyclo[2.2.1]heptane-2-carboxylic acid in step V. is performed by extracting all non-acidic organic impurities with Methyl-tert-butylether (MTBE) and by dosing the resulting solution to aqueous hydrochloric acid. Optionally, preferably in case too much (1R,2R,3R,4S)-bicyclo[2.2.1]hept-5-ene-2,3-dicarboxylic acid is present in the product, this could be removed by re-precipitation of the product.

Advantageously the isolation described above gave a high yield and good purity. Thereby, undesirable TLC (thin-layer chromatography) and/or NMR (Nuclear Magnetic Resonance Spectrometer), drying with sodium sulfate and evaporation to dryness can be avoided.

In another embodiment of the invention in step VI. the coupling of (1S,2S,4R)-bicyclo[2.2.1]heptane-2-carboxylic acid with a benzylamine, preferably 3-(pentafluoro-A6-sulfaneyl)phenyl)methanamine,) is performed using propylphosphonic anhydride (T3P) as coupling reagent and ethyl acetate as solvent.

In another embodiment of the invention the isolation of a compound according to formula I is preferably performed in step VI. Further preferred, the isolation of a compound according to formula I in step VI. is performed by basic and acidic aqueous extractions, azeotropically drying and crystallising. Preferably, the crystallisation is performed from cyclohexane or ethyl acetate and n-heptane.

Advantageously the isolation described above gave a high yield and good purity. Thereby, dichloromethane and drying with sodium sulfate and evaporation to dryness could be avoided.

In another embodiment of the invention the inventive process, in particular step I., step II., step IV. and/or step VI., is free of the use of dichloromethane.

The use of solvents such as dichloromethane is often used irrespective of their unsafe environmental characteristics.

The inventive process preferably allows reliable and safe synthetic procedures without the use of undesired solvents such as dichloromethane, by replacing dichloromethane by less harmful alternatives.

In another embodiment of the invention the inventive process, in particular step IV., is free of the use of N,N-dimethylformamide (DMF).

DMF is a harmful solvent (teratogenic, risk for formation of N-nitrosamines). Usually, no emission of DMF is allowed. As a result, this solvent should be replaced in the inventive process if possible.

Further, DMF may cause the formation of a dimethylamino impurity in chemical synthesis processes.

Preferably, in step IV. it could surprisingly be discovered, that using DMF as catalyst in the acid chloride formation is not necessary for the preparation of the acid chloride. Therefore, the ability to perform step IV. (barton decarboxylation) without DMF, limits the risk of forming N-nitrosamines.

Preferably, in step IV. the acid chloride formation stage proceeds well in toluene. Preferably, this gave the opportunity to avoid the use of dichloromethane and to submit the acid chloride to the decarboxylation step without a solvent switch in the inventive process.

Therefore, DMF can be omitted in the inventive the process, in particular in step IV.

In another embodiment of the invention the R₁ of formula I is a substituent selected from the group consisting of heteroatoms, in particular F, SF₅, CF₃ or OCF₃, or alkyl groups, in particular an ethyl-, methyl- or propyl-group.

In another embodiment of the invention formula I is selected from a group comprising
(1R,2R,4S)-rel-N-(3-(pentafluoro-λ⁶-sulfanyl)benzyl)bicyclo[2.2.1]heptane-2-carboxamide,
(1S,2S,4R)-N-(3-(pentafluoro-λ⁶-sulfanyl)benzyl)bicyclo[2.2.1]heptane-2-carboxamide,
(1R,2R,4S)-rel-N-(3-(trifluormethyl)benzyl)bicyclo[2.2.1]heptane-2-carboxamide,
(1R,2R,4S)-rel-N-(3-(trifluoromethoxy)benzyl)bicyclo[2.2.1]heptane-2-carboxamide,
(1S,2S,4R)-N-(3-(trifluoromethyl)benzyl)bicyclo[2.2.1]heptane-2-carboxamide, and
(1S,2S,4R)-N-(3-(trifluoromethoxy)benzyl)bicyclo[2.2.1]heptane-2-carboxamide.

Preferably, formula I is
(1R,2R,4S)-rel-N-(3-(pentafluoro-λ⁶-sulfanyl)benzyl)bicyclo[2.2.1]heptane-2-carboxamide,
or (1S,2S,4R)-N-(3-(pentafluoro-λ⁶-sulfanyl)benzyl)bicyclo[2.2.1]heptane-2-carboxamide,
(1S,2S,4R)-N-(3-(pentafluoro-λ⁶-sulfanyl)benzyl)bicyclo[2.2.1]heptane-2-carboxamide has the empirical formula C₁₅H₁₈F₅NOS, a MW of 355.37, the CAS number 2243284-19-5, and the formula X.

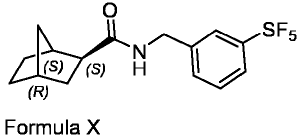

The compounds listed above belong to a class of pharmaceutically active agents which preferably function as potassium channel openers, in particular as openers of the Kv7.4 potassium channel and are therefore especially useful in the treatment of disorders associated with an aberrant potassium activity like Alzheimer's disease and Parkinson's disease. Further disorders are neurologic conditions such as epilepsy or cognitive psychiatric disorders like depression, mania and schizophrenia. In particular, it is known that potassium channels play an important role for the normal function of the outer hair cells (OHC) in the organ of the Corti of the cochlea. Therefore, the compounds listed above are promising candidates for the treatment and/or prophylaxis of hearing loss, e.g. before a treatment with an ototoxicity inducing drug.

According to a second aspect, the invention relates to a pharmaceutical composition or a medicament comprising a compound, preferably a compound according to formula I, obtained or obtainable by a process according to the first aspect of the invention.

Preferably, according to a second aspect, the invention relates to a pharmaceutical composition or a medicament comprising a compound according to formula I, preferably as described in the fourth aspect of the invention.

With respect to further features and advantages of the pharmaceutical composition or medicament reference is made in its entirety to the features and advantages described in terms of the process according to the first aspect of the invention. The features and advantages described under the first aspect of the invention do apply, mutatis mutandis, with respect to the pharmaceutical composition or medicament according to the second aspect of the invention.

According to a third aspect, the invention relates to a pharmaceutical composition or medicament according to the second aspect of the invention, for the use in the prevention or treatment of inner ear diseases. Preferably said pharmaceutical composition or said medicament is provided for transtympanic administration.

Preferably, according to a third aspect, the invention relates to a pharmaceutical composition or medicament comprising a compound according to formula I, preferably obtained by a method according to the first aspect of the invention, for the use in the prevention or treatment of inner ear diseases. Preferably, said pharmaceutical composition or said medicament is provided for transtympanic administration.

Preferably, the pharmaceutical composition or medicament is administered via transtympanic administration providing a sustained release of the pharmaceutically active agent into the ear, preferably the inner ear, for a period of at least 3 days, preferably at least 5 days, in particular after a single administration.

With respect to further features and advantages of the use in a pharmaceutical composition or a medicament reference is made in its entirety to the features and advantages described in terms of to the first aspect and the second aspect of the invention. The features and advantages described under the first and second aspect of the invention do apply, mutatis mutandis, with respect to the use according to the third aspect of the invention.

According to a fourth aspect, the invention relates to a crystalline form of a compound according to formula I, preferably obtained by a process according to the first aspect of the invention, characterized by an X-ray powder diffraction (XRPD) pattern, preferably in the 2theta, using Cu-Kα radiation comprising peaks at 9.29, 9.38, 12.29, 12.56, 14.96, 15.25, 17.04, 17.43, 19.43, 19.61, 19.88, 20.02, 20.35, 20.55, 22.82, 23.27 and 24.85.

Preferably, the above-mentioned crystalline form is the crystalline form of (1S,2S,4R)-N-(3-(pentafluoro-λ⁶-sulfaneyl)benzyl)bicyclo[2.2.1]heptane-2-carboxamide.

With respect to further features and advantages of the compound, reference is made in its entirety to the features and advantages described in terms of the process according to the first, second and third aspect of the invention. The features and advantages described under the first, second and third aspect of the invention do apply, mutatis mutandis, with respect to the use according to the fourth aspect of the invention.

Further features and advantages of the invention will become clear from the following description of preferred embodiments in form of examples in conjunction with the subject-matter of the dependent claims. The individual features can be realized either singularly or severally in combination in one embodiment of the invention. The preferred embodiments merely serve for illustration and better understanding of the invention and are not to be understood as in any way limiting the invention.

### EXAMPLE SECTION

The figures show the following:

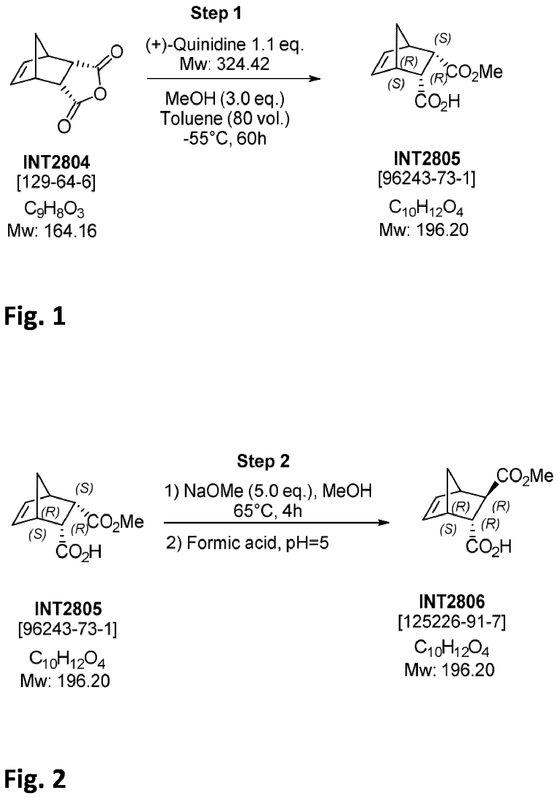

Fig.1: exemplary synthesis of step I.
Fig. 2: exemplary synthesis of step II.

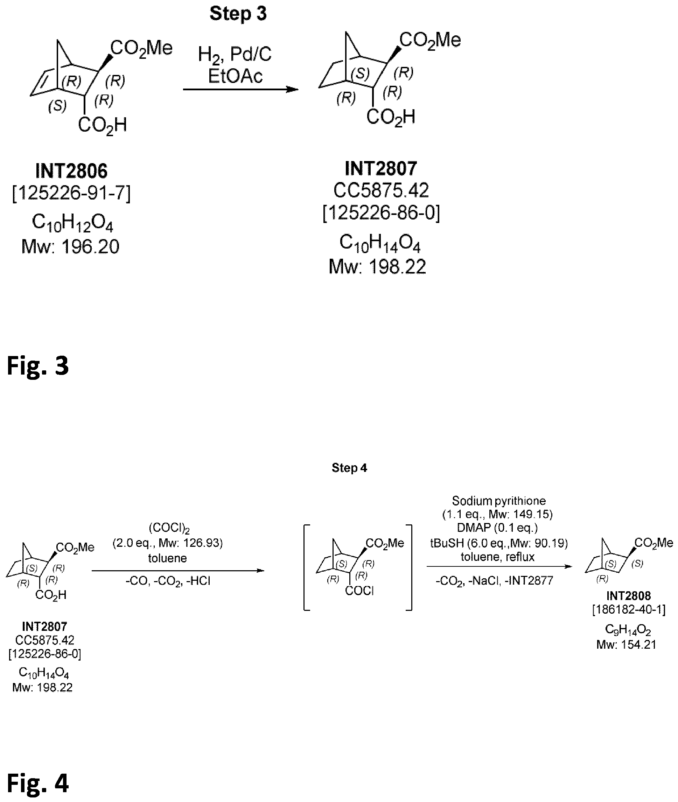

Fig. 3: exemplary synthesis of step III.
Fig. 4: exemplary synthesis of step IV.

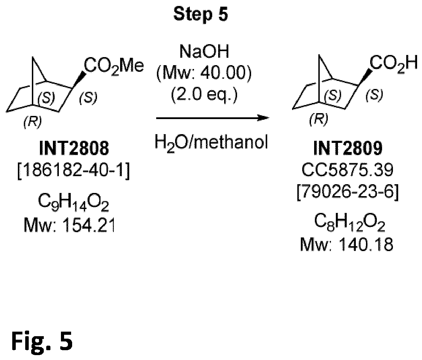

Fig. 5: exemplary synthesis of step V.

### Example of step I:

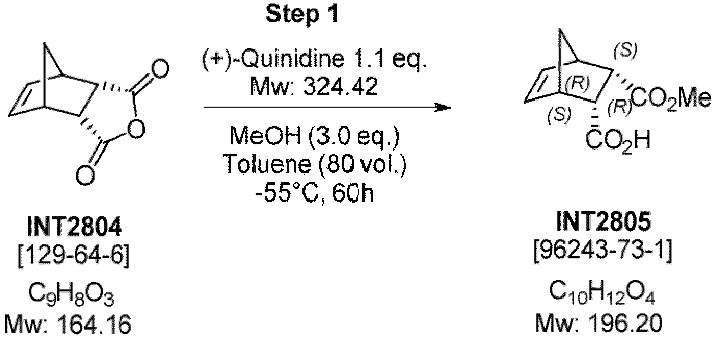

### Synthesis of step I.

1. To a 5 L nitrogen flushed reactor is charged: 43.74 g (266.4 mmoles, 1.00 eq.) carbic anhydride, preferably cis-5-Norbornene-endo-2,3-dicarboxylic anhydride or (3aR,4S,7R,7aS)-3a,4,7,7a-tetrahydro-4,7-methanoisobenzofuran-1,3-dione, 95.03 g (292.9 mmoles, 1.10 eq.) quinidine (1.1 eq.) and 3024 g (3.50 L, 80.0 rel. vol.) toluene.
2. The suspension is cooled to -60 - -55°C.
3. A solution of 25.60 g (799 mmoles, 3.00 eq.) methanol in 27.72 g (0.63 rel. wt.) toluene is dosed keeping the temperature ≤ -55.0°C. Typically, the dosing time is 15 - 30 minutes.
4. A line rinse is performed with 20 ml toluene.
5. Stirring is continued at ≤ -55.0 °C for 90-138 h. This is a possible hold point.
6. IPC-1: Conversion carbic anhydride, preferably cis-5-Norbornene-endo-2,3-dicarboxylic anhydride or (3aR,4S,7R,7aS)-3a,4,7,7a-tetrahydro-4,7-methanoisobenzofuran-1,3-dione, to (1S,2R,3S,4R)-3-(methoxycarbonyl)bicyclo[2.2.1]hept-5-ene-2-carboxylic acid should be ≥ 98.0 %. If not met, continue stirring at ≤ -55.0 °C.
7. The temperature is adjusted to 0 ± 10 °C.
8. Quinidine is extracted with 950 g (21.7 rel. wt.) + 474 g (10.8 rel. wt.) aqueous phosphoric acid (2.0 mol/kg, 19.6 wt%).
9. The product is back-extracted from the combined phosphoric acid phases with 3 x 437 ml (3 x 10.0 rel. vol.) isopropyl acetate.
10. The combined organic extracts are extracted with 450 g (10.3 rel. wt.) and 220 g (5.0 rel. wt.)10% aqueous potassium bicarbonate. This is a possible overnight hold point. Do not store the product solution for more than 21 h.
11. The combined aqueous extracts are acidified to pH= 3.5-3.7 with 19.6% aqueous phosphoric acid.
12. The acidified aqueous phase is extracted with 5 x 152 ml (5 x 3.5 rel. vol.) isopropyl acetate. Keep the pH of the aqueous phase between 3.5 and 3.7 by adding more acid if needed.
13. The combined isopropyl acetate extracts are concentrated to ± 160 ml (3.7 rel. vol.) at 150 mbar / 60°C wall temperature. After cooling to 20±5°C, this is a possible hold point.
14. 383 ml (8.8 rel. vol.) n-heptane is added.
15. 210 ml (4.8 rel. vol.) n-heptane is dosed, keeping the volume constant by distilling off solvent at 150 mbar / 60°C wall temperature. The mixture becomes slightly turbid.
16. The wall temperature is set at 45°C and the vacuum is broken.
17. The mixture is seeded with ± 10 mg solid (1S,2R,3S,4R)-3-(methoxycarbonyl)bicyclo[2.2.1]hept-5-ene-2-carboxylic acid. Crystallisation starts immediately.
18. Distilling at constant volume is resumed by dosing 175 ml (4.0 rel. vol.) n-heptane at 150 mbar / 60°C wall temperature. The internal temperature varies between 40 and 45°C.
19. The wall temperature is set at 45°C while maintaining reflux at 150 mbar at 40-45°C internal temperature.
20. The vacuum is broken, and the wall temperature is lowered from 45 to 0°C during a 90 minute period.
21. The slurry is post-stirred at 0-5°C for 3 h. This is a possible hold point.
22. The solid is filtered off, washed with 2 x 129 ml (2 x 3.0 rel. vol.) n-heptane and dried.
23. Yield: 42.37 g (81.1%) white solid with 99.3 area% LC purity and 98.5% chiral purity (97.0% e.e.).

### Example of step II.

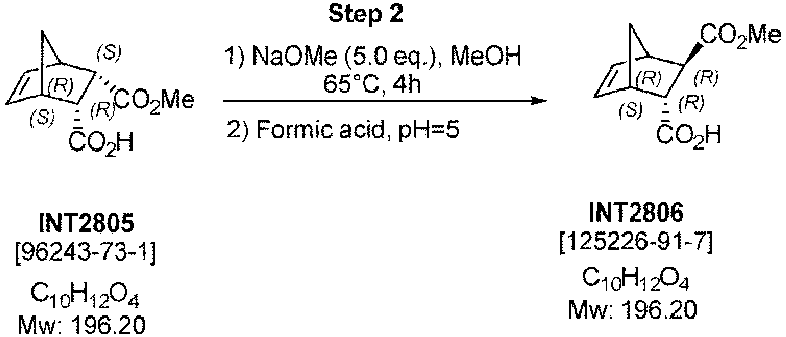

### Synthesis of step II.

1. To a 2 L nitrogen flushed reactor is charged: 30.16 g (407.5 mmoles, 1.00 eq.) methyl acetate and 570 g (720 ml, 9.0 rel. vol.) methanol.
2. 367.2 g (30% = 110.2 g, 2.04 moles, 5.00 eq.) 30% sodium methoxide in is added.
3. A line rinse is performed with 64 ml methanol.
4. The mixture is heated to 55-60°C and stirred at that temperature for at least 16 h. This is a possible hold point.
5. 80.02 g (1S,2R,3S,4R)-3-(methoxycarbonyl)bicyclo[2.2.1]hept-5-ene-2-carboxylic acid is dissolved into 240 ml (3.0 rel. vol.) methanol.
6. The temperature is adjusted to 65-70 °C.
7. The (1S,2R,3S,4R)-3-(methoxycarbonyl)bicyclo[2.2.1]hept-5-ene-2-carboxylic acid solution in methanol is dosed in a 45 - 60 minute period.
8. A line rinse is performed with 80 ml methanol.
9. After 3 h IPC: conversion is 99.0%.
10. The mixture is cooled to 20±5°C.
11. 103.1 g (2.24 moles, 5.50 eq.) formic acid is dosed. Sodium formate precipitates.
12. A line rinse is performed with 40 ml methanol. This is a possible overnight hold point.
13. Solvent is distilled off at 250 mbar / 60°C wall temperature until ± 400 ml (5.0 rel. vol.) residue.
14. 800 ml (10.0 rel. vol.) isopropyl acetate is added.
15. Solvent is distilled off at 200 mbar / 60°C wall temperature until ± 400 ml (5.0 rel. vol.) residue.
16. 400 ml (5.0 rel. vol.) demiwater is added and the mixture is stirred at high speed for 5 minutes.
17. The stirrer is stopped, and the phases are separated.
18. The upper organic phase is saved, the lower aqueous phase is extracted with 2 x 200 ml (2 x 2.5 rel. vol.) isopropyl acetate.
19. The combined isopropyl acetate extracts are separated from a small aqueous phase present and concentrated at 150 mbar 60°C wall temperature to ± 300 ml (3.75 rel. vol.). The mixture is not dry yet.
20. 200 ml (2.5 rel. vol.) isopropyl acetate is added, and the mixture is concentrated at 150 mbar / 60°C wall temperature to ± 360 ml (4.5 rel. vol.). The mixture is dry now. This is a possible overnight hold point.
21. 400 ml (5.0 rel. vol.) n-heptane is added, and the clear solution is concentrated at 150 mbar / 60°C wall temperature to ± 400 ml (5.0 rel. vol.).
22. Another 400 ml n-heptane is added, and the mixture is concentrated at 150 mbar / 60°C wall temperature to ± 200 ml (2.5 rel. vol.). Crystallisation occurs at 43°C internal temperature. Seeding may be required if crystallisation doesn't occur. A thick, difficultly stirrable slurry is obtained.
23. The slurry is vacuum cooled by setting the reactor in reflux mode and gradually reducing the pressure to 50 mbar.
24. The wall temperature is reduced to 30°C.
25. 100 ml (1.25 rel. vol.) cyclohexane is added to improve stirrability.
26. The wall temperature is lowered from 30 to 10°C during a 1 h period.
27. Post stirring is applied at 10±5°C for 2 h. This is a possible overnight hold point.
28. The solid is filtered off, washed with 2 x 100 ml (2 x 1.25 rel. vol.) n-heptane and dried under a stream of nitrogen at 20±5°C.
29. Yield: 56.01 g (70.0%) white solid with 99.0% LC purity and 99.5% chiral purity.
30. Some scaling on the reactor wall is dissolved into 150 ml (1.88 rel. vol.) cyclohexane at 70-80°C.
31. The clear solution is cooled to 20-25°C. Crystallisation occurs.
32. The solid is filtered off.
33. The mother liquor is returned into the reactor and heated to 70-80°C.
34. The clear solution is cooled to 20-25°C. Crystallisation occurs.
35. The solid is filtered off over the same filter. No washing is applied.
36. The solid is dried in the air at 20±5°C until constant weight.
37. Yield: 12.38 g (15.5%) white solid with 99.6% LC purity and 99.9% chiral purity. Total yield: 68.39 g (85.5%).

### Example of step III.

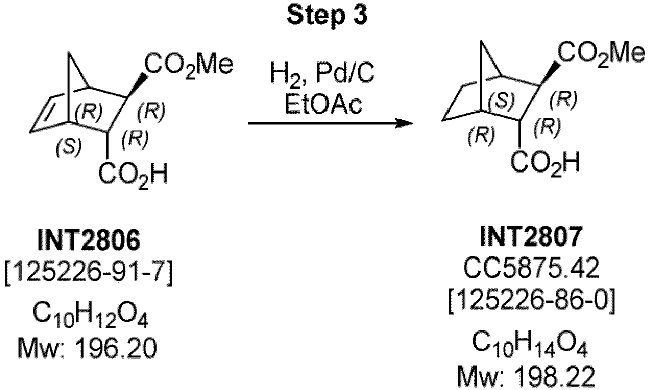

### Synthesis of step III.

1. To a 1.5 L autoclave is charged: 20.00 g (101.9 mmoles, 1.00 eq.) (1S,2R,3R,4R)-3-(methoxycarbonyl)bicyclo[2.2.1]hept-5-ene-2-carboxylic acid and 175 g (194 ml, 9.7 rel. vol.) ethyl acetate.
2. 21.3 mg (10% which contains 50% water, 1.06 mg (0.010 mmoles, 0.010 mol%) 10% palladium on charcoal (Noblyst P1070 (Evonik)) is added.
3. A line rinse is performed with 9.0 g (10 ml) ethyl acetate.
4. The autoclave is pressurised to 4.0 barg with nitrogen, after which the pressure is released. This is executed 3 times.
5. The autoclave is pressurised to 4.0 barg with hydrogen, after which the pressure is released. This is executed 3 times.
6. The stirrer is started at ± 1000 rpm and the mixture is stirred at that speed at 20-25°C at 4.0 barg hydrogen pressure for at least 2 h.
7. The autoclave is pressurised to 4.0 barg with nitrogen, after which the pressure is released. This is executed 3 times. This is a possible hold point.
8. IPC: conversion complete, no starting material detected.
9. The catalyst is filtered off.
10. The filter cake is washed with 2 x 9.0 g (2 x 10 ml) ethyl acetate.
11. The filtrate is used as is in the next step. The yield is assumed to be quantitative. Typical LC purity ± 98% (excluding dihydroquinidine residues), depending on the purity of the starting (1S,2R,3R,4R)-3-(methoxycarbonyl)bicyclo[2.2.1]hept-5-ene-2-carboxylic acid.

### Example of step IV.

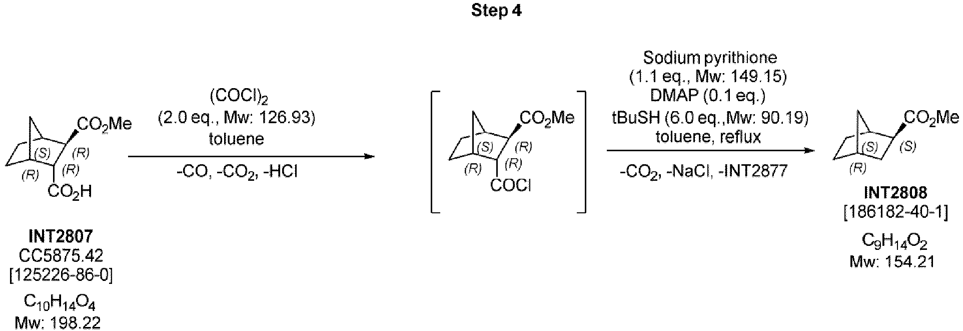

### Synthesis of step IV.

1. To a 1 L distillation flask 633.31 g (1R,2R,3R,4S)-3-(methoxycarbonyl)bicyclo[2.2.1]heptane-2-carboxylic acid solution in ethyl acetate (obtained from step 3, yield assumed 100%, 60.65 g (306.0 mmoles) (1R,2R,3R,4S)-3-(methoxycarbonyl)bicyclo[2.2.1]heptane-2-carboxylic acid) is charged.
2. The solution is concentrated to ± 150 ml (2.5 rel. vol.) at 500 mbar / 63±5°C.
3. 400 ml (6.6 rel. vol.) toluene is added.
4. The mixture is concentrated to ± 150 ml (2.5 rel. vol.) at 250 mbar / 69±5°C.
5. The concentrate is diluted with 400 ml (6.6 rel. vol.) toluene. This is a possible hold point.
6. The toluene solution is cooled to 0-5°C, after which 77.7 g (612 mmoles, 2.00 eq.) oxalyl chloride is dosed in 39 minutes. A gentle gas evolution is observed.
7. A line rinse is performed with 20 ml toluene.
8. The mixture is allowed to warm to 20-25°C and post stirred for at least 4 h. This is a possible hold point.
9. IPC shows complete conversion.
10. The mixture is concentrated to ± 200 ml (3.3 rel. vol.) at 200 mbar / 60±5°C. The excess of oxalyl chloride is removed this way.
11. The concentrate is diluted with 400 ml (6.6 rel. vol.) toluene and is stable under nitrogen at 20±5°C for 3 days. This is a possible hold point.
12. In a 3 L reactor equipped with mechanical stirrer, thermometer, dropping funnel and reflux divider with condenser, 52.91 g (assay = 95%, 50.26 g, 336.6 mmoles, 1.10 eq.) sodium pyrithione and 3.74 g (30.6 mmoles, 0.10 eq.) DMAP are suspended into 1820 ml (30.0 rel. vol.) toluene.
13. The mixture is heated to reflux while stirring at ± 400 rpm, to avoid clogging of sodium pyrithione. During heating some water droplets are observed on the wall of the flask. These droplets are distilled off together with ± 200 ml (3.3 rel. vol.) solvent.
14. The suspension is cooled to < 80°C, after which 165.6 g (1836 mmoles, 6.00 eq.) tert-butylmercaptan is dosed during 5 minutes.
15. A line rinse is performed with 50 ml toluene.
16. The mixture is heated to reflux (102-105°C).
17. The acid chloride solution from step 11 is added dropwise in 45-90 minutes. A gentle gas evolution is observed.
18. A line rinse is performed with 50 ml toluene.
19. The mixture is post stirred at reflux for at least 1 h. This is a possible hold point.
20. IPC shows 99.8% conversion.
21. The mixture is concentrated at atmospheric pressure to ± 400 ml to remove the excess of tert-butyl mercaptan (stench).
22. The solid is filtered off. The filter cake is washed with 50 ml toluene. This is a possible hold point.
23. The filtrate is extracted twice with 600 g 25% aqueous potassium carbonate.
24. The toluene phase is extracted twice with 600 g 5 N aqueous hydrochloric acid.
25. The toluene phase is washed with 600 g demiwater.
26. The toluene phase is diluted with 600 ml methanol and concentrated at atmospheric pressure to ± 200 ml.
27. The concentrate is diluted with 600 ml methanol and concentrated to ± 200 ml at atmospheric pressure.
28. Residue: 0.23 L, 188.4 g. Assay = 14.80 wt% = 27.89 g (59.1% yield, based on the intake of (1S,2R,3R,4R)-3-(methoxycarbonyl)bicyclo[2.2.1]hept-5-ene-2-carboxylic acid in the hydrogenation step) methyl (1S,2S,4R)-bicyclo[2.2.1]heptane-2-carboxylate is isolated with 84.9 area% GC purity. This material is submitted to the next step (hydrolysis) without further purification.

### Example of step V.

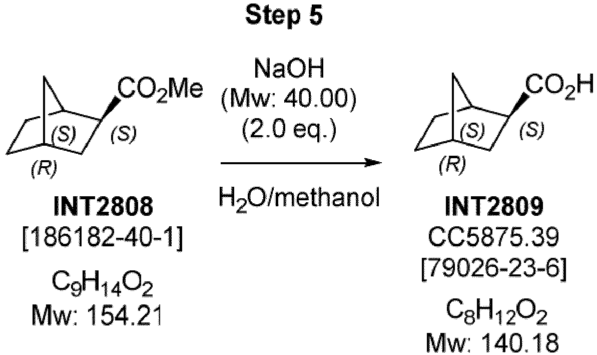

### Synthesis of step V.

1. To a 2 L jacketed reactor 188.4 g (assay = 14.80 wt%, 27.89 g, 180.9 mmoles) methyl (1S,2S,4R)-bicyclo[2.2.1]heptane-2-carboxylate in methanol is charged.
2. 265 ml (9.5 rel. vol.) methanol and 44.0 g (33% = 14.52 g = 363 mmoles, 2.01 eq.) 33% aqueous sodium hydroxide are added.
3. The mixture is stirred at 30±5°C for at least 16 h. This is a possible hold point.
4. IPC: conversion should be ≥ 99.0%, if not, the mixture post stirred at 30±5°C until conversion is met.
5. 280 g demiwater is added and the mixture is concentrated to ± 300 ml at 300 mbar / 55±2°C.
6. The concentrate is cooled to < 25°C and extracted with 3 x 280 ml MTBE.
7. Solvent residues are removed from the aqueous phase at 100 mbar / 50°C.
8. The aqueous phase is dosed to a mixture of 64 g 30% aqueous hydrochloric acid and 118 g demiwater at < 10°C. The product precipitates.
9. The slurry is post stirred at 5±5°C for at least 15 minutes. This is a possible hold point.
10. The solid is filtered off, washed with 2 x 84 g cold (0-5°C) demiwater and dried in the air at 20±5°C.
11. Yield: 24.80 g (57.8% over step 3, 4 and 5) very light beige solid with 96.3% LC purity.

### Example of step VI.

1. To a 2 L jacketed reactor 20.00 g (142.7 mmoles) (1S,2S,4R)-bicyclo[2.2.1]heptane-2-carboxylic acid and 504 g (559 ml) ethyl acetate are charged.
2. A solution of 32.29 g (138.5 mmoles, 0.97 eq.) benzylamine, preferably the benzylamine is 3-(pentafluoro-A6-sulfaneyl)phenyl)methanamine, in 108 g (120 ml) ethyl acetate is added.
3. The mixture is cooled to 0 - 5°C.
4. 31.80 g (314.4 mmoles, 2.20 eq.) N-methylmorpholine is added.
5. 118.11 g (50% = 59.06 g, 185.6 mmoles, 1.30 eq.) propylphosphonic anhydride 50% in ethyl acetate is dosed at 0 - 5°C.
6. The mixture is allowed to warm to 20 ± 5°C and post stirred for at least 16 h. This is a possible hold point.
7. IPC: conversion should be ≥ 99.0%, if not, the mixture post stirred at 20 ± 5°C until conversion is met.
8. The mixture is extracted with 400 g and 200 g 25% aqueous potassium carbonate, 200 g 1N aqueous hydrochloric acid and 200 g demiwater.
9. The ethyl acetate phase is concentrated to ± 160 ml.
10. The concentrate is diluted with 144 g (160 ml) ethyl acetate and concentrated to ± 160 ml. This is a possible hold point.
11. 125 g (160 ml) cyclohexane is added, and the mixture is concentrated to ± 160 ml. This is executed 3 times. A thick slurry results.
12. 250 g (320 ml) cyclohexane is added, and the mixture is heated to reflux to dissolve the solid present.
13. The solution is cooled to 10 ± 2°C during 35 minutes.
14. The slurry is post stirred at 10 ± 2°C for at least 1.5 h. This is a possible hold point.
15. The solid is filtered off, washed with 2 x 62 g (2 x 80 ml, 2 x 4.0 rel. vol.) cold (10 ± 2°C) cyclohexane and dried in the air at 20 ± 5°C.
16. Yield: 42.00 g (85.3%) white solid with 99.4% LC purity and 99.6% chiral purity.

## Claims

1. Process for the synthesis of a compound according to formula I or a pharmaceutically acceptable salt thereof 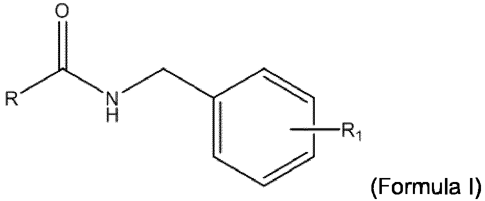 wherein
- R is a bicycloalkyl group, preferably an unsubstituted bicycloalkyl group,
comprising the following steps, preferably performed sequential in time,
I. asymmetric ring opening of a carbic anhydride, preferably cis-5-Norbornene-endo-2,3-dicarboxylic anhydride or (3aR,4S,7R,7aS)-3a,4,7,7a-tetrahydro-4,7-methanoisobenzofuran-1,3-dione, yielding (1S,2R,3S,4R)-3-(methoxycarbonyl)bicyclo[2.2.1]hept-5-ene-2-carboxylic acid,
II. epimerizing of (1S,2R,3S,4R)-3-(methoxycarbonyl)bicyclo[2.2.1]hept-5-ene-2-carboxylic acid yielding (1S,2R,3R,4R)-3-(methoxycarbonyl)bicyclo[2.2.1]hept-5-ene-2-carboxylic acid,
III. hydrogenating (1S,2R,3R,4R)-3-(methoxycarbonyl)bicyclo[2.2.1]hept-5-ene-2-carboxylic acid yielding (1R,2R,3R,4S)-3-(methoxycarbonyl)bicyclo[2.2.1]heptane-2-carboxylic acid,
IV. decarboxylating (1R,2R,3R,4S)-3-(methoxycarbonyl)bicyclo[2.2.1]heptane-2-carboxylic acid yielding methyl (1S,2S,4R)-bicyclo[2.2.1]heptane-2-carboxylate,
V. hydrolysing methyl (1S,2S,4R)-bicyclo[2.2.1]heptane-2-carboxylate yielding (1S,2S,4R)-bicyclo[2.2.1]heptane-2-carboxylic acid,
VI. coupling (1S,2S,4R)-bicyclo[2.2.1]heptane-2-carboxylic acid with a benzylamine, preferably substituted with a substituent selected from the group consisting of heteroatoms, in particular F, SF₅, CF₃ or OCF₃, or an alkyl group, in particular an ethyl-, methyl- or propyl-group, yielding a compound according to formula I, and
VII. optionally crystallising of the compound according to formula I.

2. Process according to claim 1, wherein in step I. the asymmetric ring opening of the carbic anhydride, preferably cis-5-Norbornene-endo-2,3-dicarboxylic anhydride or (3aR,4S,7R,7aS)-3a,4,7,7a-tetrahydro-4,7-methanoisobenzofuran-1,3-dione, is performed using methanol in the presence of (+)-quinidine at a temperature of -45°C to - 70°C, more preferably -50°C to -65°C, further preferred -55°C to -60°C, in toluene.

3. Process according to claim 1 or 2, wherein in step I. the product (1S,2R,3S,4R)-3-(methoxycarbonyl)bicyclo[2.2.1]hept-5-ene-2-carboxylic acid is separated from (+)-quinidine by an extraction and crystallisation comprising the following steps, preferably performed sequential in time,
a. adding of phosphoric acid to a reaction mixture of step I to dissolve (+)-quinidine in an aqueous phase,
b. back extraction of the aqueous phase with isopropyl acetate or toluene to increase the yield of the product (1S,2R,3S,4R)-3-(methoxycarbonyl)bicyclo[2.2.1]hept-5-ene-2-carboxylic acid,
c. extraction of (1S,2R,3S,4R)-3-(methoxycarbonyl)bicyclo[2.2.1]hept-5-ene-2-carboxylic acid form an organic phase into the aqueous phase with potassium bicarbonate,
d. acidification of the aqueous phase with phosphoric acid and extraction of the product into the organic phase with an organic solvent, preferably isopropyl acetate or ethyl acetate, and
e. isolation of (1S,2R,3S,4R)-3-(methoxycarbonyl)bicyclo[2.2.1]hept-5-ene-2-carboxylic acid by crystallisation from isopropyl acetate and n-heptane.

4. Process according to any of the proceeding claims, wherein in step II. the epimerizing of (1S,2R,3S,4R)-3-(methoxycarbonyl)bicyclo[2.2.1]hept-5-ene-2-carboxylic acid is performed using sodium methoxide and methanol as a solvent comprising the following steps, preferably performed sequential in time,
a. removing of possible hydroxide ions from sodium methoxide by reacting sodium methoxide, preferably with 1. eq. of methyl acetate, preferably for at least 16 h at 55 - 60°C,
b. dissolving of (1S,2R,3S,4R)-3-(methoxycarbonyl)bicyclo[2.2.1]hept-5-ene-2-carboxylic acid in methanol and adding the solution into sodium methoxide, preferably 5.0 eq. sodium methoxide, prepared in step a), and preferably reacting at 65-70°C for 3 h,
c. quenching the reaction mixture, prepared in step b), by using 4 to 6 eq. of formic acid, preferably 5.5 eq. of formic acid,
d. switching the solvent to isopropyl acetate, and extraction of the product into an organic phase with organic solvent, preferably isopropyl acetate or ethyl acetate, preferably isopropyl acetate, and
e. optionally isolation of (1S,2R,3R,4R)-3-(methoxycarbonyl)bicyclo[2.2.1]hept-5-ene-2-carboxylic acid by crystallisation from n-heptane and cyclohexane.

5. Process according to any of the proceeding claims, wherein in step III. the hydrogenating of (1S,2R,3R,4R)-3-(methoxycarbonyl)bicyclo[2.2.1]hept-5-ene-2-carboxylic acid is performed using hydrogen gas in the presence of a palladium on charcoal catalyst, present in an amount from 0,001 % to 0,1 % by weight, preferably from 0,005 % to 0,05 % by weight, more preferably from 0,0075 % to 0,0125 % by weight, based on the total weight of the composition, in ethyl acetate.

6. Process according to any of the proceeding claims, wherein in step IV. the decarboxylating of (1R,2R,3R,4S)-3-(methoxycarbonyl)bicyclo[2.2.1]heptane-2-carboxylic acid is performed using a Barton decarboxylation comprising the following steps, preferably performed sequential in time,
a. solvent switch of ethyl acetate into toluene,
b. converting (1R,2R,3R,4S)-3-(methoxycarbonyl)bicyclo[2.2.1]heptane-2-carboxylic acid into its acid chloride with oxalyl chloride and
c. decarboxylating (1R,2R,3R,4S)-3-(methoxycarbonyl)bicyclo[2.2.1]heptane-2-carboxylic acid with sodium pyrithione and tert-butyl mercaptan in 30 to 50 rel. vol, preferably 40 rel. vol., solvent, preferably toluene, at a temperature between 90°C and 120°C, preferably between 100°C and 105°C.

7. Process according to any of the proceeding claims, wherein in step V. the hydrolysing of methyl (1S,2S,4R)-bicyclo[2.2.1]heptane-2-carboxylate is performed with sodium hydroxide in methanol, preferably 0.75M to 1.25M, more preferably 1M sodium hydroxide, in methanol.

8. Process according to any of the proceeding claims, wherein in step V. isolation of (1S,2S,4R)-bicyclo[2.2.1]heptane-2-carboxylic acid is performed by extracting all non-acidic organic impurities with Methyl-tert-butylether (MTBE) and by dosing the resulting solution to aqueous hydrochloric acid.

9. Process according to any of the proceeding claims, wherein in step VI. the coupling of (1S,2S,4R)-bicyclo[2.2.1]heptane-2-carboxylic acid with a benzylamine, preferably 3-(pentafluoro-λ6-sulfaneyl)phenyl)methanamine, is performed using propylphosphonic anhydride as coupling reagent and ethyl acetate as solvent.

10. Process according to any of the proceeding claims, wherein in step VI. isolation of a compound according to formula I is performed by basic and acidic aqueous extractions, azeotropically drying and crystallising from cyclohexane.

11. Process according to any of the proceeding claims, wherein R₁ is a substituent selected from the group consisting of heteroatoms, in particular F, SF₅, CF₃ or OCF₃, or alkyl groups, in particular an ethyl-, methyl- or propyl-group.

12. Process according to any of the proceeding claims, wherein formula I is selected from a group comprising
(1R,2R,4S)-rel-N-(3-(pentafluoro-sulfanyl)benzyl)bicyclo[2.2.1]heptane-2-carboxamide,
(1S,2S,4R)-N-(3-(pentafluoro-sulfanyl)benzyl)bicyclo[2.2.1]heptane-2-carboxamide,
(1R,2R,4S)-rel-N-(3-(trifluormethyl)benzyl)bicyclo[2.2.1]heptane-2-carboxamide,
(1R,2R,4S)-rel-N-(3-(trifluoromethoxy)benzyl)bicyclo[2.2.1]heptane-2-carboxamide,
(1S,2S,4R)-N-(3-(trifluoromethyl)benzyl)bicyclo[2.2.1]heptane-2-carboxamide, and
(1S,2S,4R)-N-(3-(trifluoromethoxy)benzyl)bicyclo[2.2.1]heptane-2-carboxamide.

13. Pharmaceutical composition or medicament comprising a compound obtained by a process of any of the preceding claims.

14. Pharmaceutical composition or medicament according to claim 13, for the use in the prevention or treatment of inner ear diseases.

15. A crystalline form of a compound according to formula I, **characterized by** an X-ray powder diffraction (XRPD) pattern using Cu-Kα radiation comprising peaks at 9.29, 9.38, 12.29, 12.56, 14.96, 15.25, 17.04, 17.43, 19.43, 19.61, 19.88, 20.02, 20.35, 20.55, 22.82, 23.27 and 24.85.
